# EUROPEAN PATENT APPLICATION

(11) **EP 2 476 391 A1**
(43) Date of publication of application: **18.07.2012**
(21) Application number: 12150676.0
(22) Date of filing: 10.01.2012
(51) Int. Cl.: A61F 2/00, A61B 17/06, A61B 17/00

(54) **An implant for supporting the pelvic floor**

(30) Priority: 12.01.2011 IL 21058411
(71) Applicant: Neuman, Ilana, 99797 Carmei-Yosef (IL)
(72) Inventor: Neuman, Menahem, 99797 Carmei-Yosef (IL)
(74) Representative: Virdee-Crofts, Kulwinder Kaur

(57) **Abstract**

In one aspect, the present invention is directed to an implant (10) for supporting the pelvic floor, the implant (10) consisting essentially of (Figs. 1, 12, 16, 18): one or more straps (40, 48A, 48B, 48C) having a width of less than 25 millimeter, for anchoring the ends (86) thereof to a pelvis, thereby providing a low-mass implant (10) to avoid mesh related adverse effects.

## Description

### Field of the Invention

The present invention relates to the field of pelvic floor reconstruction. In particular, the present invention relates to the field of pelvic floor reconstruction using implants.

### Background of the Invention

Pelvic organ prolapse (POP) is a common female problem that can have a profound impact on a woman's quality of life.

The organs in the pelvic cavity, uterus, vagina, bladder and rectum, are held in place by a web of muscles and connective tissues that act much like a hammock. When these muscles and tissues become weakened or damaged, one or more of the pelvic organs shift out of normal position and literally "fall" into the vagina.

Modern prolapse surgical reconstruction is performed through the vagina. During the procedure, the surgeon repositions the prolapsed organs, securing them to surrounding tissues and ligaments.

Commonly a synthetic non-absorbable polypropylene mesh implant shaped as a plain sheath surface is used.

However, the prior art surgical procedures using mesh implants shaped as plain sheath surface do not provide reliable anchoring. One of their disadvantages is their high mass.

It is an object of the present invention to provide a reliable anchoring of the mesh implant.

Other objects and advantages of the invention will become apparent as the description proceeds.

### Summary of the Invention

The phrase "consisting essentially of" limits the scope to the specified element(s).

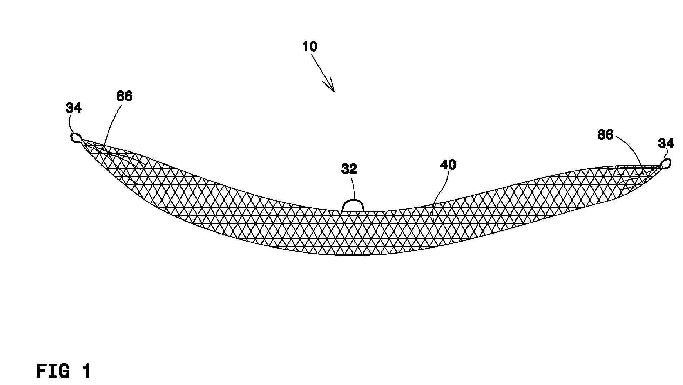

In one aspect, the present invention is directed to an implant (10) for supporting the pelvic floor, the implant (10) consisting essentially of (Figs. 1, 12, 16, 18):
- one or more straps (40, 48A, 48B, 48C) having a width of less than 25
   millimeter, for anchoring the ends (86) thereof to a pelvis,
   thereby providing a low-mass implant (10) to avoid mesh related adverse effects.
   15

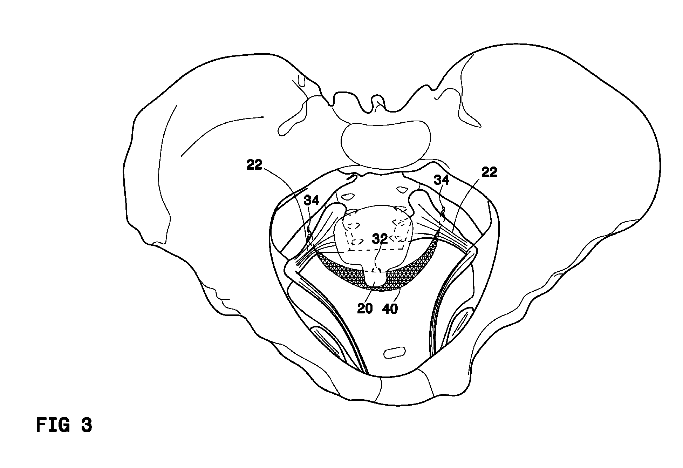

The one or more straps (40, 48A, 48B, 48C) may comprise (Figs. 1, 3):
- a mother strap (40),
wherein the length thereof is adapted to anchor, by the ends (86) thereof, the two sacrospinous ligaments (22) of the pelvis, and to support the uterine cervix (20) in between,
thereby allowing supporting the central compartment of the pelvic floor.

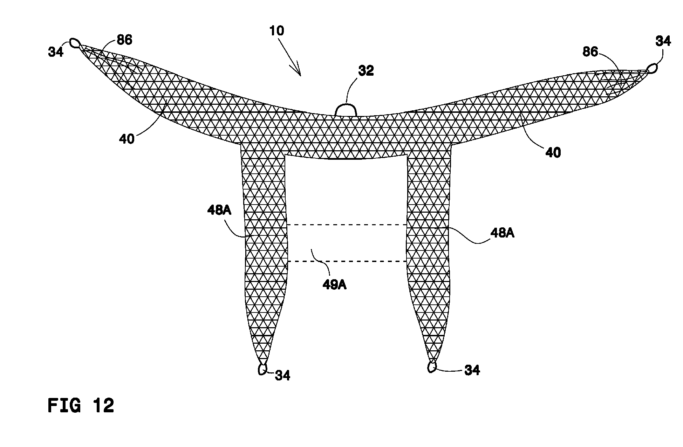

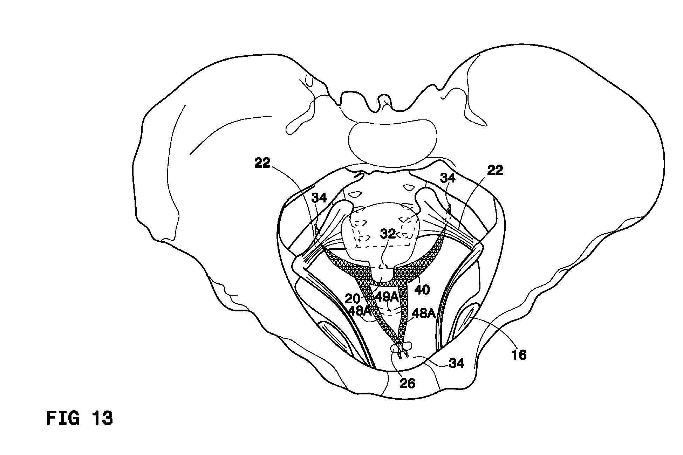

The one or more straps (40, 48A, 48B, 48C) may further comprise (Figs. 12, 13):
- a second strap (48A), extending approximately from the center of the mother strap (40), the length of the second strap (48A) thereof adapted to anchor the perineal body (26),
thereby allowing supporting the central and posterior compartments of the pelvic floor.

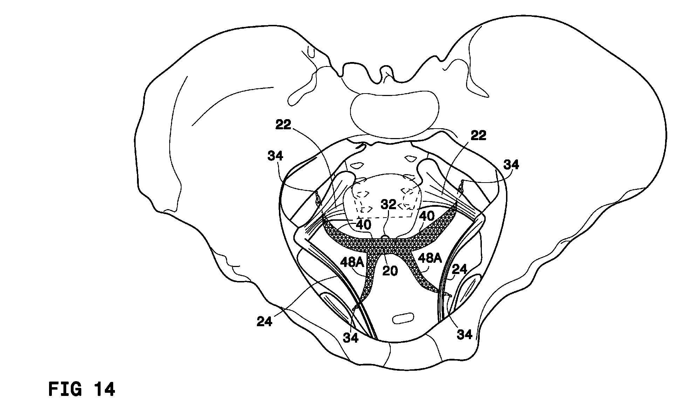

The one or more straps (40, 48A, 48B, 48C) may further comprise (Figs. 12, 14):
- a first pair of straps (48A), each strap thereof extending approximately from the center of the mother strap (40), the length of each the strap adapted to anchor by the end (86) thereof, one arcus tendineous fascia pelvic (ATFP) ligament (24),
thereby allowing supporting the central and anterior compartments of the pelvic floor.

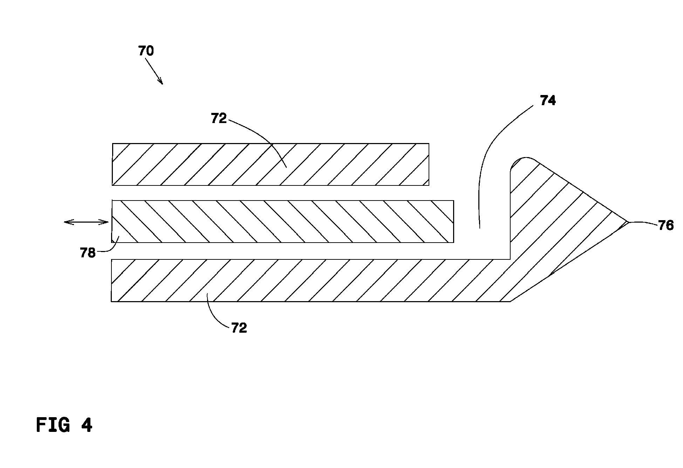

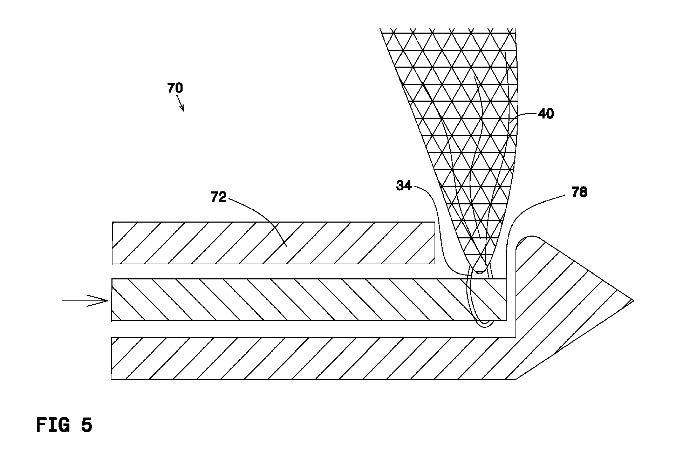

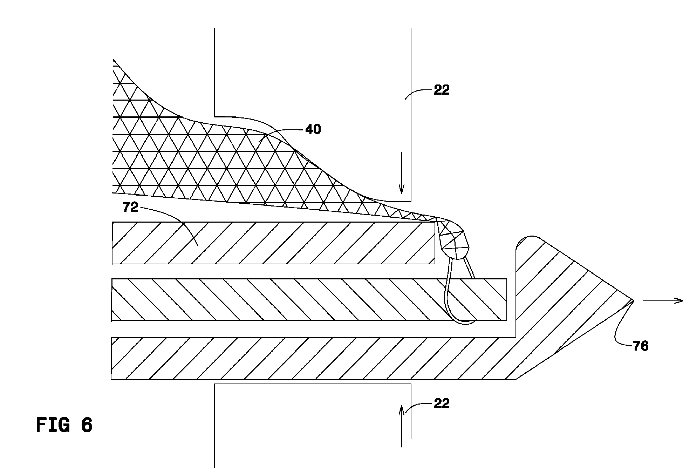

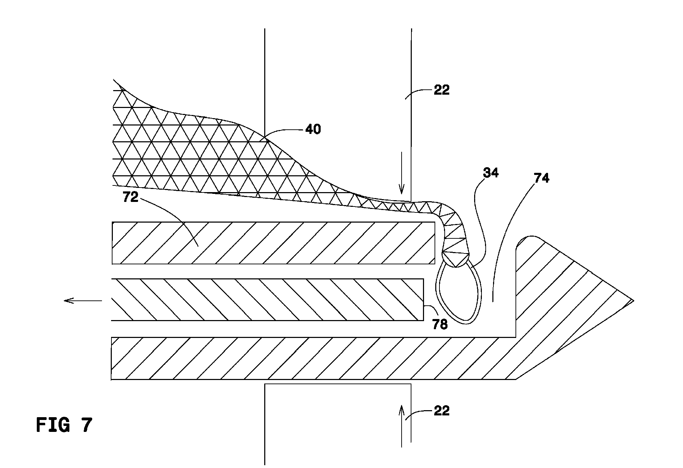

Each of the one or more straps (40, 48A, 48B, 48C) may comprise a loop (34) at the end (86) thereof, for being trapped (Figs. 4, 5) by a needle (70) and for being released (Fig. 7) within the pelvic area after threading (Fig. 6) the end (86) into a tissue (22,24, 26),
thereby allowing reliable anchoring of the end (86) to the pelvis.

The mother strap (40) may comprise a loop (32), for fixing the utero-cervical fibrotic ring (20).

The at least first pair of straps (48A) extending from the mother strap (40) may comprise (Fig. 12) a third strap (49A) in between,
thereby providing additional support.

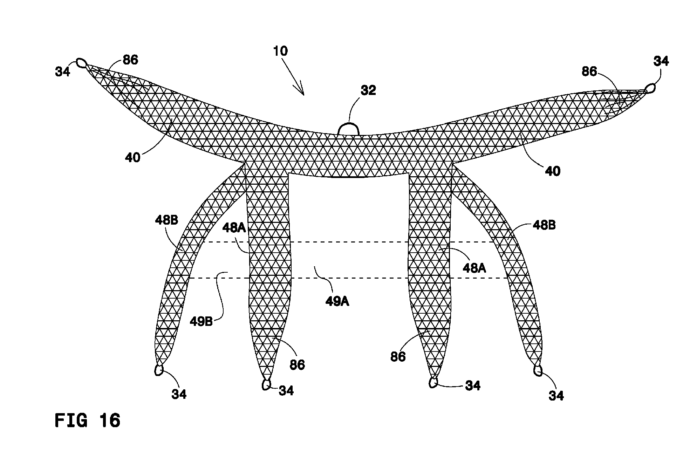

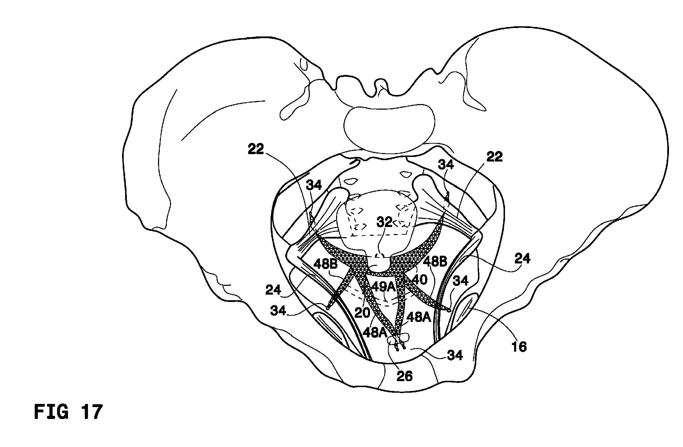

The central and anterior embodiment may further comprise (Figs. 16, 17):
- a second strap (48A), extending approximately from the center of the mother strap (40), the length of the second strap (48A) thereof adapted to anchor the perineal body (26),
thereby allowing supporting the central, posterior and anterior compartments of the pelvic floor.

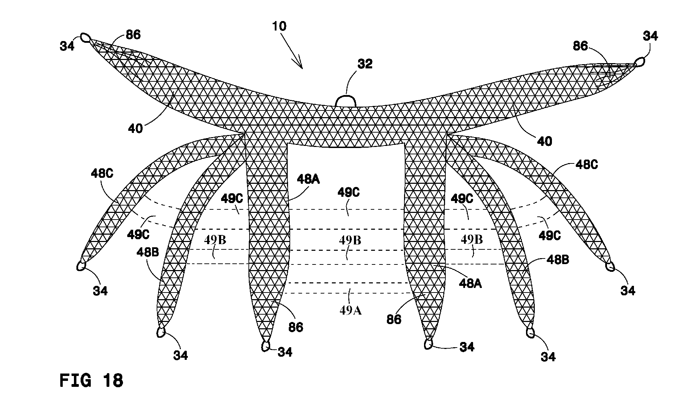

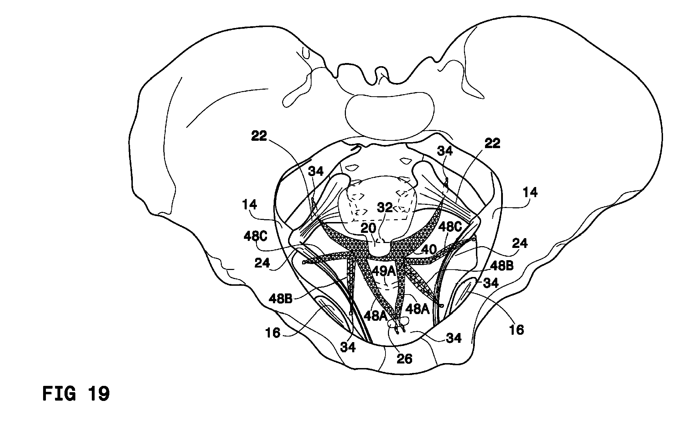

The one or more straps or straps (40, 48A, 48B, 48C) may further comprise (Fig. 18, 19):
- a second pair of straps (48C), each strap thereof extending approximately from the center of the mother strap (40), the length of each the strap adapted to anchor by the end (86) thereof, one arcus tendineous fascia pelvic (ATFP) ligament (24),
thereby providing
the first pair of straps (48A) anchoring the arcus tendineous fascia pelvic (ATFP) ligaments (24) close to the obturator foramen covered with the obturator pascia (16), and
the second pair of straps (48C) anchoring the arcus tendineous fascia pelvic (ATFP) ligaments (24) close to the iscial spine bone (14).

The reference numbers have been used to point out elements in the embodiments described and illustrated herein, in order to facilitate the understanding of the invention. They are meant to be merely illustrative, and not limiting. Also, the foregoing embodiments of the invention have been described and illustrated in conjunction with systems and methods thereof, which are meant to be merely illustrative, and not limiting.

### Brief Description of the Drawings

Embodiments and features of the present invention are described herein in conjunction with the following drawings:
FIG. 1 illustrates a mother implant, according to one embodiment of the present invention.
FIG. 2 is an anterior view of the pelvic area before installing any implant.
FIG. 3 illustrates the view of FIG. 2 after installing the implant of Fig. 1.
FIG. 4 illustrates the head of a needle for threading the ends of the implant of Fig. 1.
FIG. 5 illustrates the first step of threading the strap of the implant, using the needle of FIG. 4.
FIG. 6 illustrates the second step of threading the strap of the implant, using the needle of FIG. 4.
FIG. 7 illustrates the third step of threading the strap of the implant, using the needle of FIG. 4.
FIG. 8 illustrates the fourth step of threading the straps of the implants, using the needle of FIG. 4.
FIG. 9 illustrates the needle of FIG. 4 and its operation.
FIG. 10 illustrates the operation of the needle of FIG. 6 in respect of the surgeon's access to the pelvic area.
FIG. 11 illustrates the operation of the needle of FIG. 4 threaded into another ligament.
FIG. 12 illustrates an implant for posterior or anterior reinforcement according to another embodiment of the present invention.
FIG. 13 depicts the view of FIG. 2 after installing the implant of Fig. 12.
FIG. 14 depicts the view of FIG. 2 after installing the implant of Fig. 12, according to another embodiment.
FIG. 15 depicts the installation of Fig. 14 having the additional strap.
FIG. 16 illustrates an implant according to another embodiment of the present invention.
FIG. 17 depicts the view of FIG. 2 after installing the implant of Fig. 16.
FIG. 18 illustrates an implant according to another embodiment of the present invention.
FIG. 19 depicts the view of FIG. 2 after installing the implant of Fig. 18.

It should be understood that the drawings are not necessarily drawn to scale.

### Detailed Description of Preferred Embodiments

The present invention will be understood from the following detailed description of preferred embodiments, which are meant to be descriptive and not limiting. For the sake of brevity, some well-known features, methods, systems, procedures, components, circuits, and so on, are not described in detail.

The present invention provides an implant for supporting a female pelvic floor, having a lower mass than prior art implants. The low mass feature may avoid mesh related adverse effects.

In order to obtain this advantage, the inventive implant does not include any plain sheath surface, but rather relatively narrow straps only.

Thus, the inventive implant functions as a harness, rather than being an encasement for the supported organs.

The following text associates each strap to the pelvic support thereof, disclosing that narrow straps only may also provide sufficient and improved pelvic support.

FIG. 1 illustrates a mother implant according to one embodiment of the present invention.

An implant 10 includes a single strap 40 ending with ends 86. Implant 10 may also include a loop 32 at the center and loops 34 at the ends thereof.

Implant 10 does not include plain sheath surfaces, but only narrow strap 40 or additional straps. The width of strap 40 does not exceed 25 millimeter, thus the mass of implant 10 is reduced in relation to prior art implants.

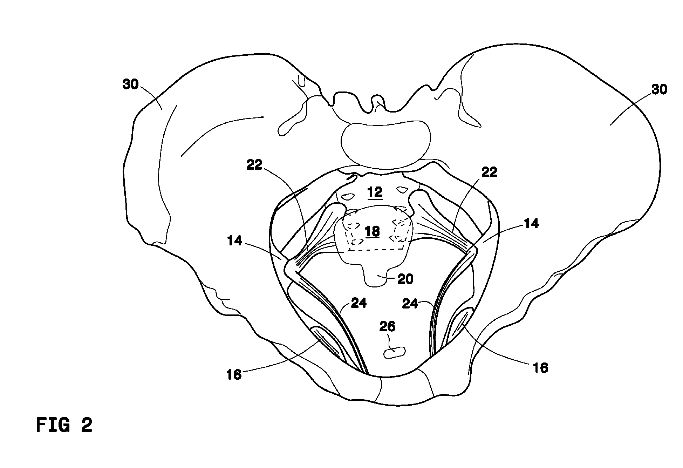

FIG. 2 is an anterior view of the pelvic area before installing any implant.

The anterior view of the pelvic area refers to the front, allowing surgical access through the patient's vaginal opening.

The ischial spine bone 14 and sacrum bone 12 are depicted extending from the posterior border of the bony pelvis 30.

The perineal body 26 is located at the posterior compartment of the pelvic area.

The uterine cervix and utero-cervical fibrotic ring 20, extending from the uterus (womb) 18, and the two ligaments of the sacrospinous (SS) 22 are located substantially at the central section of the pelvic area, at the posterior apical part of the pelvic floor connecting the sacrum and the iliac spine.

The two ligaments of the arcus tendineous fascia pelvic (ATFP) 24 are located at the anterior compartment of the pelvic area, and connect the iliac spine to the obtorator area.

FIG. 3 illustrates the view of FIG. 2 after installing the implant of Fig. 1.

Implant 10 of Fig. 1 is used for reconstructing the central section of the pelvic floor, supporting the uterine cervix and utero-cervical fibrotic ring 20.

Each of the two ends 86 of the single strap of implant 10 together with the loop 34 thereof is threaded into one sacrospinous ligament 22. Thus, each end 86 of implant 10 is anchored to one sacrospinous ligament 22.

The anchoring is adjusted such that central loop 32 of implant 10 is fixed to uterine cervix and utero-cervical fibrotic ring 20, supports it and preferably holds it. Central loop 32 of implant 10 may be sutured to uterine cervix 20 or to the location vaginal apex and/or naturally adhered after long term attachment, and/or immediately adhered by a biological adhesive, glue, tuckers, sutures, or just placing and depending upon natural adhesion, or any other attachment.

Since the mass of strap 40 is smaller than prior art mesh implants, the anchoring thereof is safer and less related to adverse effects.

FIG. 4 illustrates the head of a needle for threading the ends of the implant of Fig. 1.

A needle 70 may be used for threading ends 86 of implant 10 or other ends of other implants through ligaments 22 and/or through other places.

The head of needle 70 includes a rod 78, which may be manually slid back and forth within a body 72.

The edge of rod 78 may be inserted into a niche 74. The tip 76 of needle 70 is located at the edge of body 72.

FIG. 5 illustrates the first step of threading the strap of the implant, using the needle of FIG. 4.

The surgeon inserts loop 34 of strap 40 into niche 74, and traps the edge of rod 78 into loop 34.

FIG. 6 illustrates the second step of threading the strap of the implant, using the needle of FIG. 4.

The surgeon then pushes tip 76 together with body 72 into ligament 22 or into another ligament, threading loop 34 and strap 30 therethrough.

FIG. 7 illustrates the third step of threading the strap of the implant, using the needle of FIG. 4.

The surgeon then slides rod 78 out of niche 74, releasing loop 34.

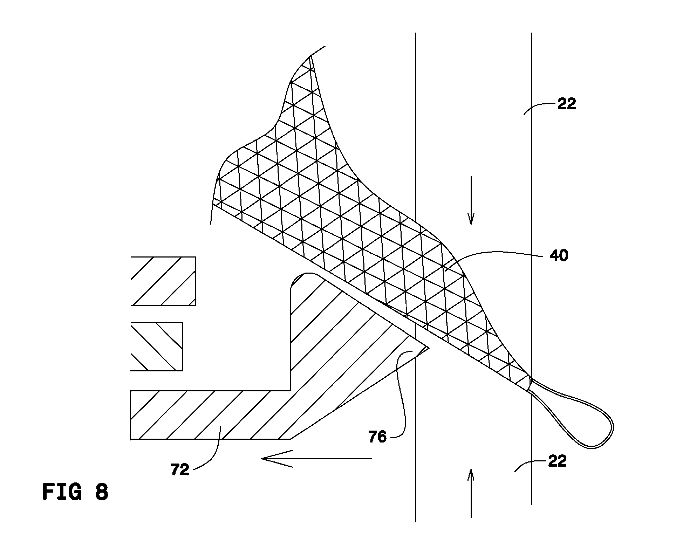

FIG. 8 illustrates the fourth step of threading the straps of the implants, using the needle of FIG. 4.

The surgeon then pulls body 72, together with tip 76, out of ligament 22. Strap 40 remains threaded while tip 76 exits.

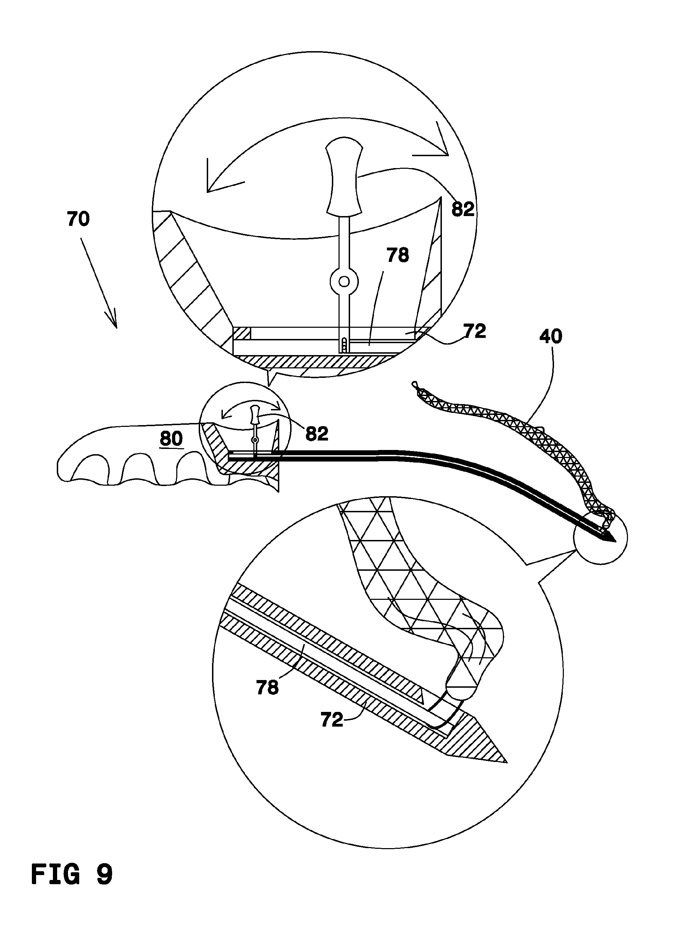

FIG. 9 illustrates the needle of FIG. 4 and its operation.

The surgeon holds handle 80 of needle 70, and slides rod 78 by toggling a toggle arm 82, which is connected to rod 78.

Since tip 76 is inserted into the pelvic area, and toggle arm 82 is far away from tip 76, toggle arm 82 is located outside the body of the patient and may be located farther and outside the surgical area.

Thus, the surgeon can thread strap 40 from the side having surgical access, without requiring any additional perforations of the body from the opposing direction.

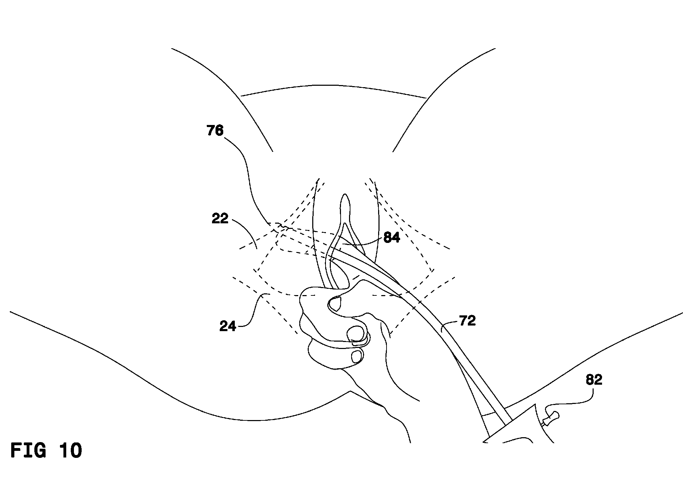

FIG. 10 illustrates the operation of the needle of FIG. 6 in respect of the surgeon's access to the pelvic area.

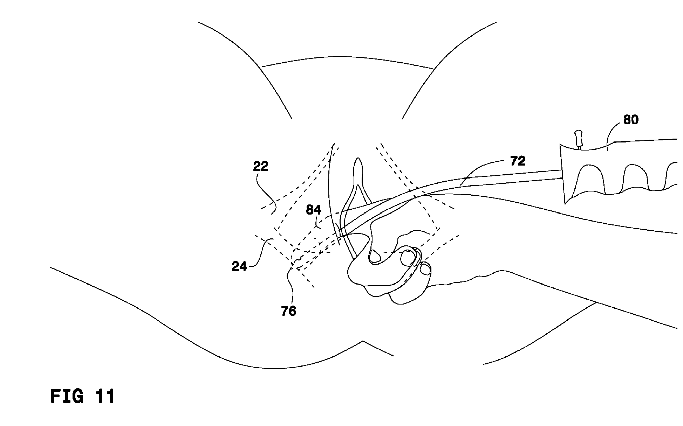

FIG. 11 illustrates the operation of the needle of FIG. 4 threaded into another ligament.

The surgeon inserts finger 84 thereof in vaginal incision into the pelvic area (the lines of the parts inside are dashed). The surgeon may trap loop 34 to niche 74 of needle 70, then penetrate tip 76 through ligament 22 or 24 and pushes into the desired depth; then releases loop 34 from needle 70 by toggling toggle arm 82, using the other hand thereof. The surgeon can then pull tip 76 back, leaving loop 34 and strap 30 at the side beyond, based on the shrinking of ligament 22 or 24 towards strap 30 at the threaded point.

FIG. 12 illustrates an implant for posterior or anterior reinforcement according to another embodiment of the present invention.

Implant 10 according to this embodiment includes, except for strap 40 ending with ends 86 and loop 32 at the center, also two additional straps 48A, each extending substantially perpendicularly from strap 40.

Since straps 48A and other straps extend from strap 40, strap 40 is herein named "mother strap".

Each of straps 48A may also include a loop 34 at the end thereof.

According to one embodiment, an additional strap 49A may cross between the perpendicular straps 48A, for limiting the distance therebetween.

FIG. 13 depicts the view of FIG. 2 after installing the implant of Fig. 12 on the central and posterior compartments.

Implant 10 of Fig. 12 is used for reconstructing the central and the posterior compartment of the pelvic floor, supporting the uterine cervix and utero-cervical fibrotic ring 20 and/or the location vaginal apex, the small intestine and the rectum.

Each of the two ends 86 of the mother strap 40 together with the loop 34 thereof is threaded into one sacrospinous ligament 22. Implant 10 is then adjusted between sacrospinous ligaments 22 such that loop 32 of implant 10 thereof supports and preferably holds the uterine cervix and utero-cervical fibrotic ring 20 and/or the location vaginal apex.

Loop 32 at the center of implant 10 may be sutured to uterine cervix 20 and/or to the location vaginal apex and/or naturally adhered after long term attachment, and/or immediately adhered by a biological adhesive, glue, tuckers, sutures, or just placing and depending upon natural adhesion, or any other attachment.

Each of perpendicular straps 48A is then extended to the perineal body 26 at the back, for supporting also the posterior compartment of the pelvic floor, the small intestine and the rectum.

According to one embodiment, loops 34 of straps 48A are threaded through perineal body 26. According to another embodiment, loops 34 may be sutured to perineal body 26 and/or naturally adhered by long term attachment, and/or adhered by a biological adhesive.

FIG. 14 depicts the view of FIG. 2 after installing the implant of Fig. 12 on the central and anterior compartments.

According to this embodiment, implant 10 of Fig. 12 may also be used for reconstructing the central and the anterior compartment of the pelvic floor, supporting the uterine cervix and utero-cervical fibrotic ring 20 and/or the location vaginal apex, and the urinary bladder.

Like Fig. 13, each of the two ends 86 of mother strap 40 together with the loop 34 thereof is threaded into one sacrospinous ligament 22. Implant 10 is then adjusted between sacrospinous ligaments 22 such that central loop 32 of implant 10 thereof supports and preferably holds the uterine cervix and utero-cervical fibrotic ring 20 and/or the location vaginal apex. Central loop 32 of implant 10 may be sutured to uterine cervix 20 and/or to the location vaginal apex, and/or naturally adhered by long term attachment, and/or adhered by a biological adhesive.

Unlike Fig. 13, each of extending straps 48A is then extended to be threaded into one arcus tendineous fascia pelvic (ATFP) ligament 24 and/or the obturator foramen covered with obturator pascia 16 for supporting the anterior compartment of the pelvic floor and the urinary bladder.

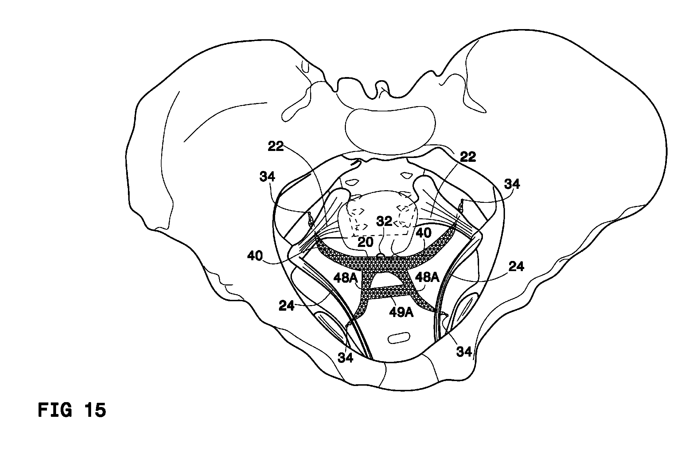

FIG. 15 depicts the installation of Fig. 14 on the central and anterior compartments, having the additional strap.

Additional strap 49A crossing between the perpendicular straps 48A, for limiting the distance therebetween, may provide further support to the anterior compartment of the pelvic floor and the urinary bladder.

FIG. 16 illustrates an implant according to another embodiment of the present invention.

Implant 10 according to this embodiment includes the straps of Fig. 12, namely mother strap 40 and additional straps 48A extending substantially perpendicularly from mother strap 40. Implant 10 may include additional strap 49A crossing between the perpendicular straps 48A.

Implant 10 of this embodiment may include in addition to implant 10 of Fig. 12 straps 48B, which are similar to straps 48A. An additional strap 49B may cross between the perpendicular straps 48B, for limiting the distance therebetween.
FIG. 17 depicts the view of FIG. 2 after installing the implant of Fig. 16 on the central, posterior and anterior compartments.
Fig. 17 combines Figs. 13 and 14.

Implant 10 of Fig. 16 is used for reconstructing the central and the posterior compartment of the pelvic floor, supporting the uterine cervix and utero-cervical fibrotic ring 20 and/or the location vaginal apex, the small intestine and the rectum, and also the anterior compartment of the pelvic floor and the urinary bladder.

Each of the two ends 86 of the mother strap 40 together with the loop 34 thereof is threaded into one sacrospinous ligament 22. Implant 10 is then adjusted between sacrospinous ligaments 22 such that central loop 32 of implant 10 thereof supports and preferably holds the central section of the pelvic floor, the uterine cervix and utero-cervical fibrotic ring 20 and/or the location vaginal apex.

Each of perpendicular straps 48A is then extended to the perineal body 26 at the back, for also supporting the posterior compartment of the pelvic floor, the small intestine and the rectum.

Each of extending straps 48B is then extended to be threaded into one arcus tendineous fascia pelvic (ATFP) ligament 24 and/or the obturator foramen covered with obturator pascia 16 for supporting the anterior compartment of the pelvic floor and the urinary bladder.

FIG. 18 illustrates an implant according to another embodiment of the present invention.

Implant 10 according to this embodiment includes the straps of Fig. 16, namely mother strap 40 and additional straps 48A and 48B, extending substantially perpendicularly from mother strap 40. Implant 10 may include additional straps 49A crossing between the perpendicular straps 48A, and additional strap 49B crossing between the perpendicular straps 48B.

Implant 10 of this embodiment may include in addition to implant 10 of Fig. 16 also straps 48C which are similar to straps 48A. An additional strap 49C may cross between the perpendicular straps 48C, for limiting the distance therebetween.

FIG. 19 depicts the view of FIG. 2 after installing the implant of Fig. 18 on the central, posterior and anterior compartments.

Straps 48B and 48C support the anterior compartment. Straps 48B may be threaded into the arcus tendineous fascia pelvic (ATFP) ligaments 24 close to the obturator foramen covered with obturator pascia 16, and straps 48C of Fig. 18 may be threaded into the arcus tendineous fascia pelvic (ATFP) ligaments 24 close to the iscial spine bone 14.

It may be noted that in Fig. 14 like in Fig. 19 the anterior compartment may also be supported by two pairs of straps.

In the figures and/or description herein, the following reference numerals have been mentioned:
- numeral 10 denotes an implant according to one embodiment of the present invention;
- numeral 12 denotes the sacrum bone;
- numeral 14 denotes the ischial spine bone;
- numeral 16 denotes the obturator foramen covered with obturator pascia;
- numeral 18 denotes the uterus (womb);
- numeral 20 denotes the uterine cervix and utero-cervical fibrotic ring;
- numeral 22 denotes a sacrospinous (SS) ligament;
- numeral 24 denotes an arcus tendineous fascia pelvic (ATFP) ligament;
- numeral 26 denotes the perineal body;
- numeral 30 denotes the bony pelvis;
- numeral 32 denotes a loop at the center of the central strap;
- numeral 34 denotes a loop at the end of any of the straps of the implant;
- numeral 40 denotes the central strap of the implant, named mother strap;
- numerals 48A, 48B and 48C denote straps extending from the central strap;
- numeral 49A denotes a strap between a pair of 48A straps;
- numeral 49B denotes a strap between a pair of 48B straps;
- numeral 49C denotes a strap between a pair of 48C straps;
- numeral 70 denotes a needle;
- numeral 72 denotes the body of the needle;
- numeral 74 denotes a niche within the needle;
- numeral 76 denotes the tip of the needle;
- numeral 78 denotes a rod within the needle;
- numeral 80 denotes the handle of the needle;
- numeral 82 denotes a toggle arm for moving the rod of the needle;
- numeral 84 denotes the finger of the surgeon; and
- numeral 86 denotes an end of the strap of the implant.

The foregoing description and illustrations of the embodiments of the invention has been presented for the purposes of illustration. It is not intended to be exhaustive or to limit the invention to the above description in any form.

Any term that has been defined above and used in the claims, should to be interpreted according to this definition.

The reference numbers in the claims are not a part of the claims, but rather used for facilitating the reading thereof. These reference numbers should not be interpreted as limiting the claims in any form.

## Claims

1. An implant (10) for supporting a female pelvic floor, said implant (10) consisting essentially of (Figs. 1, 12, 16, 18):
- one or more straps (40, 48A, 48B, 48C) having a width of less than 25
millimeters, for anchoring the ends (86) thereof to a pelvis,
thereby providing a low-mass implant (10) to avoid mesh related adverse effects.

2. An implant (10) according to claim 1, wherein said one or more straps (40, 48A, 48B, 48C) comprise (Figs. 1,3):
- a mother strap (40),
wherein the length thereof is adapted to anchor, by the ends (86) thereof, the two sacrospinous ligaments (22) of said pelvis, and to support the uterine cervix (20) in between,
thereby allowing supporting the central compartment of the pelvic floor.

3. An implant (10) according to claim 2, wherein said one or more straps (40, 48A, 48B, 48C) further comprise (Figs. 12, 13):
- at least a second strap (48A), extending approximately from the center of said mother strap (40), the length of said at least second strap (48A) thereof adapted to anchor the perineal body (26),
thereby allowing supporting the central and posterior compartments of the pelvic floor.

4. An implant (10) according to claim 2, wherein said one or more straps (40, 48A, 48B, 48C) further comprise (Figs. 12, 14):
- a first pair of straps (48A), each strap thereof extending approximately from the center of said mother strap (40), the length of each said strap adapted to anchor by the end (86) thereof, one arcus tendineous fascia pelvic (ATFP) ligament (24),
thereby allowing supporting the central and anterior compartments of the pelvic floor.

5. An implant (10) according to claim 1, wherein each of said one or more straps (40, 48A, 48B, 48C) comprises a loop (34) at the end (86) thereof, for being trapped (Figs. 4,5) by a needle (70) and for being released (Fig. 7) within the pelvic area after threading (Fig. 6) said end (86) into a tissue (22,24, 26), thereby allowing reliable anchoring of said end (86) to said pelvis.

6. An implant (10) according to claim 2, wherein said mother strap (40) comprises a loop (32), for fixing the utero-cervical fibrotic ring (20) thereto.

7. An implant (10) according to claim 4, wherein said at least first pair of straps (48A) extending from said mother strap (40) comprises (Fig. 12) a third strap (49A) in between,
thereby providing additional support.

8. An implant (10) according to claim 4, wherein said one or more straps (40, 48A, 48B, 48C) further comprise (Figs. 16, 17):
- at least a second strap (48A), extending approximately from the center of said mother strap (40), the length of said at least second strap (48A) thereof adapted to anchor the perineal body (26),
thereby allowing supporting the central, posterior and anterior compartments of the pelvic floor.

9. An implant (10) according to claim 4, wherein said one or more strap or straps (40, 48A, 48B, 48C) further comprise (Fig. 18, 19):
- a second pair of straps (48C), each strap thereof extending approximately from the center of said mother strap (40), the length of each said strap adapted to anchor by the end (86) thereof, one arcus tendineous fascia pelvic (ATFP) ligament (24),
thereby providing
said first pair of straps (48A) anchoring the arcus tendineous fascia pelvic (ATFP) ligaments (24) close to the obturator foramen covered with the obturator pascia (16), and
said second pair of straps (48C) anchoring the arcus tendineous fascia pelvic (ATFP) ligaments (24) close to the iscial spine bone (14).

10. An implant (10) according to claim 3, wherein said at least second strap (48A) comprises a pair of straps.
